# EUROPÄISCHE PATENTSCHRIFT

(11) **EP 1 242 039 B1**
(45) Veröffentlichungstag und Bekanntmachung des Hinweises auf die Patenterteilung: **16.02.2005**
(21) Anmeldenummer: 00991245.2
(22) Anmeldetag: 21.12.2000
(51) Int. Cl.: A61K 7/13, A61K 7/135, A61K 7/09, A61K 7/06

(54) **VERRINGERUNG DER HAARSCHÄDIGUNG BEI OXIDATIVEN PROZESSEN**
REDUCING DAMAGE TO HAIR DURING OXIDATIVE PROCESSES
DIMINUTION DE L'ALTERATION DES CHEVEUX DUE AUX PROCESSUS D'OXYDATION

(30) Priorität: 24.12.1999 DE 19962869
(43) Veröffentlichungstag der Anmeldung: 25.09.2002
(73) Patentinhaber: Henkel Kommanditgesellschaft auf Aktien, 40589 Düsseldorf-Holthausen (DE)
(72) Erfinder: HÖFFKES, Horst, 40595 Düsseldorf (DE); MEINIGKE, Bernd, 51381 Leverkusen (DE); OBERKOBUSCH, Doris, 40591 Düsseldorf (DE); HOLLENBERG, Detlef, 40699 Erkrath (DE); KLEEN, Astrid, 40699 Erkrath (DE)
(86) Internationale Anmeldenummer: PCT/EP2000/013087
(87) Internationale Veröffentlichungsnummer: WO 2001/047486

(56) Entgegenhaltungen:
- EP-A- 0 919 220
- EP-A- 0 965 324
- WO-A-00/59457
- WO-A-00/76469
- WO-A-94/16672
- WO-A-99/11227
- DE-A- 3 814 685
- DE-A- 4 126 429
- DE-A- 19 835 055
- US-A- 4 507 278
- US-A- 5 053 051
- US-A- 5 599 531
- US-A- 5 866 107

## Beschreibung

Die vorliegende Erfindung betrifft die Verwendung von gegebenenfalls hydratisierten SiO₂-Verbindungen zur Verringerung der Schädigung keratinischer Fasern durch oxidative Prozesse, Mittel, die diese Verbindungen in Kombination mit strukturverbessernden Wirkstoffen enthalten, sowie entsprechende Verfahren zur oxidativen Haarbehandlung.

Menschliches Haar wird heute in vielfältiger Weise mit haarkosmetischen Zubereitungen behandelt. Dazu gehören etwa die Reinigung der Haare mit Shampoos, die Pflege und Regeneration mit Spülungen und Kuren sowie das Bleichen, Färben und Verformen der Haare mit Färbemitteln, Tönungsmitteln, Wellmitteln und Stylingpräparaten. Dabei spielen Mittel zur Veränderung oder Nuancierung der Farbe des Kopfhaares eine herausragende Rolle.

Für dauerhafte, intensive Färbungen mit entsprechenden Echtheitseigenschaften werden sogenannte Oxidationsfärbemittel verwendet. Solche Färbemittel enthalten üblicherweise Oxidationsfarbstoff-vorprodukte, sogenannte Entwicklerkomponenten und Kupplerkomponenten. Die Entwicklerkomponenten bilden unter dem Einfluß von Oxidationsmitteln untereinander oder unter Kupplung mit einer oder mehreren Kupplerkomponenten die eigentlichen Farbstoffe aus. Die Oxidationsfärbemittel zeichnen sich durch hervorragende, lang anhaltende Färbeergebnisse aus.

Ferner werden häufig aufhellende Verfahren, die sogenannten Blondierverfahren, angewendet. Die Grundlagen der Blondierverfahren sind dem Fachmann bekannt und in einschlägigen Monographien, z.B. von K. Schrader, Grundlagen und Rezepturen der Kosmetika, 2. Auflage, 1989, Dr. Alfred Hüthig Verlag, Heidelberg, oder W. Umbach (Hrg.), Kosmetik, 2. Auflage, 1995, Georg Thieme Verlag, Stuttgart, New York, zusammenfassend beschrieben.

Zum Blondieren menschlicher Haare - insbesondere für die Strähnchenapplikation - werden üblicherweise feste oder pastenförmige Zubereitungen mit festen Oxidationsmitteln unmittelbar vor der Anwendung mit einer verdünnten Wasserstoffperoxidlösung vermischt. Diese Mischung wird dann auf das Haar aufgebracht und nach einer bestimmten Einwirkzeit wieder ausgespült.

Die genannten Zubereitungen, die vor der Anwendung üblicherweise mit einer Wasserstoffperoxidlösung vermischt werden, werden im weiteren als "Blondiermittel" bezeichnet. Alle aufgeführten Mengenangaben beziehen sich, soweit nicht anders ausgeführt, ausschließlich auf diese Zubereitungen.

Durch den erforderlichen Gehalt an Oxidationsmitteln strapazieren derartige Verfahren die Fasern. In einigen Fällen kann es sogar, insbesondere bei den Blondierverfahren, zu nachhaltigen Schädigungen der Fasern kommen. Es hat daher in der Vergangenheit nicht an Versuchen gefehlt, den negativen Einfluß der Oxidationsmittel auf die Fasern zu verringern. Eine Entwicklungsrichtung ist die Optimierung der in den Färbeverfahren eingesetzten Farbstoffe. So wurden beispielsweise Farbstoffe entwickelt, die bereits durch Luftsauerstoff oxidiert werden können und daher den Einsatz weiterer Oxidationsmittel überflüssig machen. Eine weitere Entwicklungsrichtung ist der Einsatz von Enzymen oder Metallsalzen als Oxidationskatalysatoren, um auf diese Weise den erforderlichen Gehalt an Oxidationsmittel zu senken. Ein Nachteil dieser Färbeverfahren ist allerdings der Umstand, daß die natürlichen Farbpigmente der Haare nicht aufgehellt werden. Das hat zur Folge, daß nur Nuancen zugänglich sind, die dunkler sind als die ursprüngliche Haarfarbe. Es wurde daher stets nach Verbindungen gesucht, die die schädigende Wirkung der Oxidationsmittel verringern oder die entstandenen Schädigungen zumindest teilweise beheben können.

Es wurde nun überraschenderweise gefunden, daß Haarschädigungen durch oxidative Prozesse durch die Verwendung spezieller SiO₂-Verbindungen deutlich vermindert werden können.

Ein erster Gegenstand der vorliegenden Erfindung ist daher die Verwendung von gegebenenfalls hydratisierten SiO₂-Verbindungen zur Verringerung der Schädigung keratinischer Fasern, insbesondere menschlicher Haare, durch oxidative Prozesse.

Unter keratinischen Fasern werden erfindungsgemäß Pelze, Wolle, Federn und insbesondere menschliche Haare verstanden.

Hinsichtlich der gegebenenfalls hydratisierten SiO₂-Verbindungen unterliegt die vorliegende Erfindung prinzipiell keinen Beschränkungen. Bevorzugt sind Kieselsäuren, deren Oligomeren und Polymeren sowie deren Salze. Bevorzugte Salze sind die Alkalisalze, insbesondere die Kalium und Natriumsalze. Die Natriumsalze sind ganz besonders bevorzugt.

Die gegebenenfalls hydratisierten SiO₂-Verbindungen können in verschiedenen Formen vorliegen. Erfindungsgemäß bevorzugt werden die SiO₂-Verbindungen in Form von Kieselgelen (Silicagel) oder als Wasserglas eingesetzt.

Erfindungsgemäß besonders bevorzugt sind Wassergläser, die aus einer wäßrigen Lösung eines Silicates der Formel (SiO₂)ₙ(Na₂O)ₘ(K₂O)ₚ gebildet werden, wobei n steht für eine positive rationale Zahl und m und p stehen unabhängig voneinander für eine positive rationale Zahl oder für 0, mit den Maßgaben, daß mindestens einer der Parameter m oder p von 0 verschieden ist und das Verhältnis zwischen n und der Summe aus m und p zwischen 1:4 und 4:1 liegt.

Neben den durch die Summenformel beschriebenen Komponenten können die Wassergläser in geringen Mengen noch weitere Zusatzstoffe, wie beispielsweise Phosphate oder Magnesiumsalze, enthalten.

Erfindungsgemäß besonders bevorzugte Wassergläser werden unter anderem von der Fa. Henkel unter den Bezeichnungen Ferrosil® 119, Natronwasserglas 40/42, Portil® A, Portil® AW und Portil® W und von der Firma Akzo unter der Bezeichnung Britesil® C20 vertrieben.

Obwohl bereits geringe Mengen der gegebenenfalls hydratisierten SiO₂-Verbindungen die Haarschädigung verringern, kann es erfindungsgemäß bevorzugt sein, die gegebenenfalls hydratisierten SiO₂-Verbindungen in Mengen von 0,05 Gew.-% bis 5 Gew.-%, besonders bevorzugt in Mengen von 0,15 Gew.-% bis 2 Gew.-% und ganz besonders bevorzugt in Mengen von 0,2 Gew.-% bis 1,5 Gew.-%, jeweils bezogen auf die gesamte Anwendungszubereitung, einzusetzen. Die Mengenangaben geben dabei jeweils den Gehalt der SiO₂-Verbindungen (ohne Wasseranteil) in den Anwendungszubereitungen wieder.

Unter oxidativen Prozessen werden erfindungsgemäß alle Verfahren verstanden, bei denen die Fasern mit einem Oxidationsmittel behandelt werden. Beispiele für derartige Verfahren stellen die Blondierung, die oxidative Färbung sowie die Fixierung im Rahmen einer Dauerwellbehandlung dar.

In einer bevorzugten Ausführungsform der vorliegenden Erfindung ist der oxidative Prozeß eine Blondierung.

Blondiermittel enthalten üblicherweise als erste wichtige Komponente Wasserstoffperoxid oder eine feste Anlagerungsverbindung von Wasserstoffperoxid an anorganische oder organische Verbindungen, wie beispielsweise Natriumperborat, Natriumpercarbonat, Natriumpercarbamid, Harnstoffperoxid und Melaminperoxid. Die Blondierwirkung kann durch sogenannte "Booster" gesteigert werden. Dies sind in der Regel feste Peroxoverbindung, die keine Anlagerungsprodukte von Wasserstoffperoxid an andere Komponenten darstellen. Die Auswahl dieser Peroxoverbindung unterliegt prinzipiell keinen Beschränkungen; übliche, dem Fachmann bekannte Peroxoverbindungen sind beispielsweise Ammoniumperoxidisulfat, Kaliumperoxidisulfat, Natriumperoxidisulfat, Ammoniumpersulfat, Kaliumpersulfat, Natriumpersulfat, Kaliumperoxidiphosphat, Percarbonate wie Magnesiumpercarbonat und Peroxide wie Bariumperoxid. Unter diesen Peroxoverbindungen, die auch in Kombination eingesetzt werden können, sind erfindungsgemäß die anorganischen Verbindungen bevorzugt. Besonders bevorzugt sind die Peroxidisulfate, insbesondere Ammoniumperoxidisulfat. Besonders bei der Verwendung von "Boostern", die sich leicht in höheren Konzentrationen in den fertigen Zubereitungen lösen, wie beispielsweise Ammoniumperoxidisulfat, ist aber die Gefahr einer verstärkten Haarschädigung gegeben.

Die Peroxoverbindungen sind in den erfindungsgemäßen Blondiermitteln bevorzugt in Mengen von 5-30 Gew.-%, insbesondere in Mengen von 8-20 Gew.-%, enthalten.

Als weitere wichtige Komponente enthalten Blondiermittel ein Alkalisierungsmittel, das zur Einstellung des alkalischen pH-Wertes der Anwendungsmischung dient. Erfindungsgemäß können die dem Fachmann ebenfalls für Blondiermittel bekannten, üblichen Alkalisierungsmittel wie Ammonium-, Alkalimetall- und Erdalkalimetallhydroxide, -carbonate, -hydrogencarbonate, -hydroxycarbonate, -silikate, insbesondere -metasilikate, sowie Alkaliphosphate verwendet werden. In einer bevorzugten Ausführungsform enthalten die erfindungsgemäßen Blondiermittel mindestens zwei unterschiedliche Alkalisierungsmittel. Dabei können Mischungen beispielsweise aus einem Metasilikat und einem Hydroxycarbonat bevorzugt sein.

Blondiermittel enthalten Alkalisierungsmittel bevorzugt in Mengen von 1-25 Gew.-%, insbesondere 1,5-20 Gew.-%.

Für die Anwendung der Mittel auf dem Haar werden die Blondiermittel unmittelbar vor dem Auftragen mit einer Wasserstoffperoxid-Lösung vermischt. Die Konzentration dieser Wasserstoffperoxid-Lösung wird einerseits von den gesetzlichen Vorgaben und andererseits von dem gewünschten Effekt bestimmt; in der Regel werden 6- bis 12-prozentige Lösungen in Wasser verwendet. Die Mengenverhältnisse von Blondiermittel und Wasserstoffperoxid-Lösung liegen dabei üblicherweise im Bereich 1:1 bis 1:2, wobei ein Überschuß an Wasserstoffperoxid-Lösung insbesondere dann gewählt wird, wenn keine zu ausgeprägte Blondierwirkung erwünscht ist.

In einer zweiten bevorzugten Ausführungsform der vorliegenden Erfindung ist der oxidative Prozeß eine oxidative Haarfärbung. Oxidative Haarfärbemittel enthalten als Vorprodukte für Oxidationsfarbstoffe sogenannte Entwickler- und Kupplerkomponenten. Die Entwicklerkomponenten bilden unter dem Einfluß von Oxidationsmitteln, gegebenenfalls mit Hilfe spezieller Enzyme oder Metallionen als Katalysator, untereinander oder unter Kupplung mit einer oder mehreren Kupplerkomponenten die eigentlichen Farbstoffe aus.

Erfindungsgemäß bevorzugte Entwicklerkomponenten sind p-Phenylendiamin, p-Toluylendiamin, p-Aminophenol, o-Aminophenol, 1-(2'-Hydroxyethyl)-2,5-diaminobenzol, N,N-Bis-(2-hydroxy-ethyl)-p-phenylendiamin, 2-(2,5-Diaminophenoxy)-ethanol, 4-Amino-3-methylphenol, 2,4,5,6-Tetraaminopyrimidin, 2-Hydroxy-4,5,6-triaminopyrimidin, 4-Hydroxy-2,5,6-triaminopyrimidin, 2,4-Dihydroxy-5,6-diaminopyrimidin, 2-Dimethylamino-4,5,6-triaminopyrimidin, 2-Hydroxymethylamino-4-amino-phenol, Bis-(4-aminophenyl)amin, 4-Amino-3-fluorphenol, 2-Aminomethyl-4-aminophenol, 2-Hydroxymethyl-4-aminophenol, 4-Amino-2-((diethylamino)-methyl)-phenol, Bis-(2-hydroxy-5-aminophenyl)-methan, 1,4-Bis-(4-aminophenyl)-diazacycloheptan, 1,3-Bis(N(2-hydroxyethyl)-N(4-aminophenylamino))-2-propanol, 4-Amino-2-(2-hydroxyethoxy)-phenol, 1,10-Bis-(2,5-diaminophenyl)-1,4,7,10-tetraoxadecan sowie 4,5-Diaminopyrazol-Derivate nach EP 0 740 931 B1 bzw. WO 94/08970 A1 wie z.B. 4,5-Diamino-1-(2'-hydroxyethyl)-pyrazol.

Erfindungsgemäß besonders bevorzugt sind die Entwicklerkomponenten p-Phenylendiamin, p-Toluylendiamin, p-Aminophenol, 1-(2'-Hydroxyethyl)-2,5-diaminobenzol, N,N-Bis-(2-hydroxy-ethyl)-p-phenylendiamin, 4-Amino-3-methylphenol, 4-Amino-2-((diethylamino)-methyl)-phenol, 2-Aminomethyl-4-aminophenol, 2,4,5,6-Tetraaminopyrimidin, 2-Hydroxy-4,5,6-triaminopyrimidin, 4-Hydroxy-2,5,6-triaminopyrimidin und 4,5-Diamino-1-(2'-hydroxyethyl)-pyrazol.

Erfindungsgemäß bevorzugte Kupplerkomponenten sind
- m-Aminophenol und dessen Derivate wie beispielsweise 5-Amino-2-methylphenol, 3-Amino-2-chlor-6-methylphenol, 2-Hydroxy-4-aminophenoxyethanol, 2,6-Dimethyl-3-aminophenol, 5-Amino-4-chlor-2-methylphenol, 5-Amino-4-methoxy-2-methylphenol, 5-(2'-Hydroxyethyl)-amino-2-methylphenol, 3-(Diethylamino)-phenol, N-Cyclopentyl-3-aminophenol, 1,3-Dihydroxy-5-(methylamino)-benzol, 3-(Ethylamino)-4-methylphenol und 2,4-Dichlor-3-aminophenol,
- o-Aminophenol und dessen Derivate,
- m-Diaminobenzol und dessen Derivate wie beispielsweise 2,4-Diaminophenoxyethanol, 1,3-Bis-(2,4-diaminophenoxy)-propan, 1-Methoxy-2-amino-4-(2'-hydroxyethylamino)benzol, 1,3-Bis-(2,4-diaminophenyl)-propan, 2,6-Bis-(2-hydroxyethylamino)-1-methylbenzol und 1-Amino-3-bis-(2'-hydroxyethyl)-aminobenzol,
- o-Diaminobenzol und dessen Derivate wie beispielsweise 3,4-Diaminobenzoesäure und 2,3-Diamino-1-methylbenzol,
- Di- beziehungsweise Trihydroxybenzolderivate wie beispielsweise Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol,
- Pyridinderivate wie beispielsweise 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin und 3,5-Diamino-2,6-dimethoxypyridin,
- Naphthalinderivate wie beispielsweise 1-Naphthol, 2-Methyl-1-naphthol, 2-Hydroxymethyl-1-naphthol, 2-Hydroxyethyl-1-naphthol, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin,
- Morpholinderivate wie beispielsweise 6-Hydroxybenzomorpholin und 6-Aminobenzomorpholin,
- Chinoxalinderivate wie beispielsweise 6-Methyl-1,2,3,4-tetrahydrochinoxalin,
- Pyrazolderivate wie beispielsweise 1-Phenyl-3-methylpyrazol-5-on,
- Indolderivate wie beispielsweise 4-Hydroxyindol, 6-Hydroxyindol und 7-Hydroxyindol und
- Methylendioxybenzolderivate wie beispielsweise 1-Hydroxy-3,4-methylendioxybenzol, 1-Amino-3,4-methylendioxybenzol und 1-(2'-Hydroxyethyl)-amino-3,4-methylendioxybenzol.

Erfindungsgemäß besonders bevorzugte Kupplerkomponenten sind 1-Naphthol, 1,5-, 2,7-und 1,7-Dihydroxynaphthalin, 3-Aminophenol, 5-Amino-2-methylphenol, 2-Amino-3-hydroxypyridin, Resorcin, 4-Chlorresorcin, 2-Chlor-6-methyl-3-aminophenol, 2-Methylresorcin, 5-Methylresorcin, 2,5-Dimethylresorcin und 2,6-Dihydroxy-3,4-dimethylpyridin.

Ferner können die oxidativen Färbemittel zur weiteren Nuancierung verschiedene direktziehende Farbstoffe enthalten.

Direktziehende Farbstoffe sind üblicherweise Nitrophenylendiamine, Nitroaminophenole, Azofarbstoffe, Anthrachinone oder Indophenole. Bevorzugte direktziehende Farbstoffe sind die unter den internationalen Bezeichnungen bzw. Handelsnamen HC Yellow 2, HC Yellow 4, HC Yellow 5, HC Yellow 6, Basic Yellow 57, HC Orange 1, Disperse Orange 3, HC Red 1, HC Red 3, HC Red 13, HC Red BN, Basic Red 76, HC Blue 2, HC Blue 12, Disperse Blue 3, Basic Blue 7, Basic Blue 26, Basic Blue 99, HC Violet 1, Disperse Violet 1, Disperse Violet 4, Basic Violet 2, Basic Violet 14, Acid Violet 43, Disperse Black 9, Acid Black 52, Basic Brown 16 und Basic Brown 17 bekannten Verbindungen sowie 1,4-Bis-(β-hydroxyethyl)-amino-2-nitrobenzol, 3-Nitro-4-(β-hydroxyethyl)-aminophenol, 4-Amino-2-nitrodiphenylamin-2'-carbonsäure, 6-Nitro-1,2,3,4-tetrahydrochinoxalin, 2-Hydroxy-1,4-naphthochinon, Hydroxyethyl-2-nitro-toluidin, Pikraminsäure, 2-Amino-6-chloro-4-nitrophenol, 4-Ethylamino-3-nitrobenzoesäure und 2-Chloro-6-ethylamino-1-hydroxy-4-nitrobenzol. Die erfindungsgemäßen Mittel gemäß dieser Ausführungsform enthalten die direktziehenden Farbstoffe bevorzugt in einer Menge von 0,01 bis 20 Gew.-%, bezogen auf das gesamte Färbemittel.

Weiterhin können die Färbezubereitungen auch in der Natur vorkommende Farbstoffe wie beispielsweise Henna rot, Henna neutral, Henna schwarz, Kamillenblüte, Sandelholz, schwarzen Tee, Faulbaumrinde, Salbei, Blauholz, Krappwurzel, Catechu, Sedre und Alkannawurzel enthalten.

Weitere in Färbemitteln enthaltene Farbstoffkomponenten können auch Indole und Indoline, sowie deren physiologisch verträgliche Salze, sein. Bevorzugte Beispiele sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 3,6-Dihydroxyindol-2-carbonsäure, 6-Hydroxyindol, 6-Aminoindol und 4-Aminoindol. Weiterhin bevorzugt sind 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindolin-2-carbonsäure, 6-Hydroxyindolin, 6-Aminoindolin und 4-Aminoindolin.

Die Indolin- und Indol-Derivate können sowohl als freie Basen als auch in Form ihrer physiologisch verträglichen Salze mit anorganischen oder organischen Säuren, z. B. der Hydrochloride, der Sulfate und Hydrobromide, eingesetzt werden.

Bei der Verwendung von Farbstoff-Vorstufen vom Indolin- oder Indol-Typ kann es bevorzugt sein, diese zusammen mit mindestens einer Aminosäure und/oder mindestens einem Oligopeptid einzusetzen. Bevorzugte Aminosäuren sind Aminocarbonsäuren, insbesondere α-Aminocarbonsäuren und ω-Aminocarbonsäuren. Unter den α-Aminocarbonsäuren sind wiederum Arginin, Lysin, Ornithin und Histidin besonders bevorzugt. Eine ganz besonders bevorzugte Aminosäure ist Arginin, insbesondere in freier Form, aber auch als Hydrochlorid eingesetzt.

Oxidative Haarfärbemittel werden üblicherweise schwach sauer bis alkalisch, d. h. auf pH-Werte im Bereich von etwa 5 bis 11, eingestellt. Zu diesem Zweck enthalten die Färbemittel Alkalisierungsmittel, üblicherweise Alkali- oder Erdalkalihydroxide, Ammoniak oder organische Amine. Bevorzugte Alkalisierungsmittel sind Monoethanolamin, Monoisopropanolamin, 2-Amino-2-methyl-propanol, 2-Amino-2-methyl-1,3-propandiol, 2-Amine-2-ethyl-1,3-propandiol, 2-Amino-2-methylbutanol und Triethanolamin sowie Alkali- und Erdalkalimetallhydroxide. Insbesondere Monoethanolamin, Triethanolamin sowie 2-Amino-2-methyl-propanol und 2-Amino-2-methyl-1,3-propandiol sind im Rahmen dieser Gruppe bevorzugt. Auch die Verwendung von ω-Aminosäuren wie ω-Aminocapronsäure als Alkalisierungsmittel ist möglich.

Zur Ausbildung der eigentlichen Haarfarben durch einen oxidativen Prozess können übliche Oxidationsmittel, wie insbesondere Wasserstoffperoxid oder dessen Anlagerungsprodukte an Harnstoff, Melamin oder Natriumborat verwendet werden. Die Oxidation mit Luftsauerstoff als einzigem Oxidationsmittel kann allerdings bevorzugt sein. Weiterhin ist es möglich, die Oxidation mit Hilfe von Enzymen durchzuführen, wobei die Enzyme sowohl zur Erzeugung von oxidierenden Per-Verbindungen eingesetzt werden als auch zur Verstärkung der Wirkung einer geringen Menge vorhandener Oxidationsmittel. So können die Enzyme (Enzymklasse 1: Oxidoreduktasen) Elektronen aus geeigneten Entwicklerkomponenten (Reduktionsmittel) auf Luftsauerstoff übertragen. Bevorzugt sind dabei Oxidasen wie Tyrosinase und Laccase aber auch Glucoseoxidase, Uricase oder Pyruvatoxidase. Weiterhin sei das Vorgehen genannt, die Wirkung geringer Mengen (z. B. 1% und weniger, bezogen auf das gesamte Mittel) Wasserstoffperoxid durch Peroxidasen zu verstärken.

Zweckmäßigerweise wird die Zubereitung des Oxidationsmittels dann unmittelbar vor dem Färben der Haare mit der Zubereitung mit den Farbstoffvorprodukten vermischt. Das dabei entstehende gebrauchsfertige Haarfärbepräparat sollte bevorzugt einen pH-Wert im Bereich von 6 bis 10 aufweisen. Besonders bevorzugt ist die Anwendung der Haarfärbemittel in einem schwach alkalischen Milieu. Die Anwendungstemperaturen können in einem Bereich zwischen 15 und 40 °C, bevorzugt bei der Temperatur der Kopfhaut, liegen. Nach einer Einwirkungszeit von ca. 5 bis 45, insbesondere 15 bis 30, Minuten wird das Haarfärbemittel durch Ausspülen von dem zu färbenden Haar entfernt. Das Nachwaschen mit einem Shampoo entfällt, wenn ein stark tensidhaltiger Träger, z. B. ein Färbeshampoo, verwendet wurde.

Insbesondere bei schwer färbbarem Haar kann die Zubereitung mit den Farbstoffvorprodukten ohne vorherige Vermischung mit der Oxidationskomponente auf das Haar aufgebracht werden. Nach einer Einwirkdauer von 20 bis 30 Minuten wird dann - gegebenenfalls nach einer Zwischenspülung - die Oxidationskomponente aufgebracht. Nach einer weiteren Einwirkdauer von 10 bis 20 Minuten wird dann gespült und gewünschtenfalls nachshampooniert. Bei dieser Ausführungsform wird gemäß einer ersten Variante, bei der das vorherige Aufbringen der Farbstoffvorprodukte eine bessere Penetration in das Haar bewirken soll, das entsprechende Mittel auf einen pH-Wert von etwa 4 bis 7 eingestellt. Gemäß einer zweiten Variante wird zunächst eine Luftoxidation angestrebt, wobei das aufgebrachte Mittel bevorzugt einen pH-Wert von 7 bis 10 aufweist. Bei der anschließenden beschleunigten Nachoxidation kann die Verwendung von sauer eingestellten Peroxidisulfat-Lösungen als Oxidationsmittel bevorzugt sein.

Unabhängig davon, welches der oben genannten Vorgehen im Rahmen des erfindungsgemäßen Verfahrens gewählt wird, kann die Ausbildung der Färbung dadurch unterstützt und gesteigert werden, daß dem Mittel bestimmte Metallionen zugesetzt werden. Solche Metallionen sind beispielsweise Zn²⁺, Cu²⁺, Fe²⁺, Fe³⁺, Mn²⁺, Mn⁴⁺, Li⁺, Mg²⁺, Ca²⁺ und Al³⁺. Besonders geeignet sind dabei Zn²⁺, Cu²⁺ und Mn²⁺. Die Metallionen können prinzipiell in der Form eines beliebigen, physiologisch verträglichen Salzes eingesetzt werden. Bevorzugte Salze sind die Acetate, Sulfate, Halogenide, Lactate und Tartrate. Durch Verwendung dieser Metallsalze kann sowohl die Ausbildung der Färbung beschleunigt als auch die Farbnuance gezielt beeinflußt werden.

Ein zweiter Gegenstand der vorliegenden Erfindung sind Mittel zur Behandlung keratinischer Fasern, enthaltend ein Oxidationsmittel, mindestens eine gegebenenfalls hydratisierte SiO₂-Verbindung in Form von Kieselgelen (Silicagel) oder als Wasserglas und mindestens einen strulcturverbessernden Wirkstoff, der ausgewählt ist aus Panthenol, physiologisch verträglichen Panthenol-Derivaten, Mono-, Di- und Oligosacchariden, Serin, Niacinamid, Polyvinylpyrrolidon, Gluconsäure, Biotin und den lipidlöslichen Esteralkoholen oder Esterpolyolen.

Hinsichtlich der gegebenenfalls hydratisierten SiO₂-Verbindungen sowie der erfindungsgemäß bevorzugten Oxidationsmittel sei an dieser Stelle explizit auf die weiter oben dargelegten Ausführungen verwiesen.

Die gegebenenfalls hydratisierten SiO₂-Verbindungen sind in den erfindungsgemäßen Mittel bevorzugt in Mengen von mehr als 0,05 Gew.-%, insbesondere mehr als 0,3 Gew.-%, enthalten.

Bevorzugte, strukturverbessernde Wirkstoffe stellen Panthenol und seine physiologisch verträglichen Derivate dar. Solche Derivate sind insbesondere die Ester und Ether des Panthenols sowie kationisch derivatisierte Panthenole. Einzelne Vertreter sind beispielsweise das Panthenoltriacetat, der Panthenolmonoethylether und dessen Monoacetat sowie die in der WO 92/13829 A1 offenbarten kationischen Panthenolderivate. Ein erfindungsgemäß bevorzugtes Panthenolderivat ist ferner dessen Vorstufe Pantolacton. Panthenol ist innerhalb dieser Gruppe bevorzugt.

Weiterhin ist auch Polyvinylpyrrolidon (PVP) für seine faserstrukturverbessernden Eigenschaften bekannt und erfindungsgemäß bevorzugt.

Es können darüber hinaus zusätzlich weitere strukturverbessernde Verbindungen eingesetzt werden.

Besonders wirksame, strukturverbessernde Verbindungen stellen die Aldehyde dar. Besonders bevorzugte Beispiel sind Formaldehyd und Formaldehyd-abspaltende Verbindungen, wie beispielsweise Methoxymethylester, Dimethylol (thio) harnstoffderivate, Oxazolidinderivate, N-Hydroxymethylmaleinimid, Hexamethylentetramin und seine Derivate, Hydantoinderivate, Pyridinium-substituierte Dimethylether, Imidazolidinylharnstoff-Derivate, Isothiazolinone, 2-Brom-2-nitropropandiol und 5-Brom-5-nitro-1,3-dioxan. Weitere besonders bevorzugte Aldehyde sind Acetaldehyd, Glyoxal, Glycerinaldehyd und Glutardialdehyd.

Weiterhin können Vitamine, wie z.B. Pyroxidin (Vitamin B6) eingesetzt werden.

Eine weitere geeignete Gruppe von strukturverbessernden Wirkstoffen sind Pflanzenextrakte.

Üblicherweise werden diese Extrakte durch Extraktion der gesamten Pflanze hergestellt. Es kann aber in einzelnen Fällen auch bevorzugt sein, die Extrakte ausschließlich aus Blüten und/oder Blättern der Pflanze herzustellen.

Hinsichtlich der erfindungsgemäß verwendbaren Pflanzenextrakte wird insbesondere auf die Extrakte hingewiesen, die in der auf Seite 44 der 3. Auflage des Leitfadens zur Inhaltsstoffdeklaration kosmetischer Mittel, herausgegeben vom Industrieverband Körperpflege- und Waschmittel e.V. (IKW), Frankfurt, beginnenden Tabelle aufgeführt sind.

Erfindungsgemäß sind vor allem die Extrakte aus Eichenrinde, Brennessel, Hamamelis, Hopfen, Kamille, Klettenwurzel, Schachtelhalm, Weißdorn, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, grünem Tee, Ginseng und Ingwerwurzel bevorzugt.

Besonders bevorzugt sind die Extrakte aus Eichenrinde, Brennessel, Hamamelis, Hopfen, Kamille, Klettenwurzel, Schachtelhalm, Lindenblüten, Mandel, Aloe Vera, Kokosnuß, Mango, Aprikose, Limone, Weizen, Kiwi, Melone, Orange, Grapefruit, Salbei, Rosmarin, Birke, Wiesenschaumkraut, Quendel, Schafgarbe, Hauhechel, Meristem, grünem Tee, Ginseng und Ingwerwurzel.

Ganz besonders für die erfindungsgemäßen Mittel geeignet sind die Extrakte aus Mandel, Aloe Vera, Kokosnuß, Mango, Aprikose, Limone, Weizen, Kiwi, Melone und grünem Tee.

Als Extraktionsmittel zur Herstellung der genannten Pflanzenextrakte können Wasser, Alkohole sowie deren Mischungen verwendet werden. Unter den Alkoholen sind dabei niedere Alkohole wie Ethanol und Isopropanol, insbesondere aber mehrwertige Alkohole wie Ethylenglykol und Propylenglykol, sowohl als alleiniges Extraktionsmittel als auch in Mischung mit Wasser, bevorzugt. Pflanzenextrakte auf Basis von Wasser/Propylenglykol im Verhältnis 1:10 bis 10:1 haben sich als besonders geeignet erwiesen.

Die Pflanzenextrakte können erfindungsgemäß sowohl in reiner als auch in verdünnter Form eingesetzt werden. Sofern sie in verdünnter Form eingesetzt werden, enthalten sie üblicherweise ca. 2 - 80 Gew.-% Aktivsubstanz und als Lösungsmittel das bei ihrer Gewinnung eingesetzte Extraktionsmittel oder Extraktionsmittelgemisch.

Weiterhin kann es bevorzugt sein, in den erfindungsgemäßen Mitteln Mischungen aus mehreren, insbesondere aus zwei, verschiedenen Pflanzenextrakten einzusetzen.

Ebenfalls erfindungsgemäß als strukturverbessernde Wirkstoffe bevorzugt sind Honigextrakte. Diese Extrakte werden in analoger Weise zu den Pflanzenextrakten gewonnen und enthalten üblicherweise 1 - 10 Gew.-%, insbesondere 3 - 5 Gew.-%, Aktivsubstanz. Wasser/Propylenglykol-Mischungen können auch hier bevorzugte Extraktionsmittel sein.

Weitere strukturverbessernde Wirkstoffe sind Proteinhydrolysate, insbesondere Elastin-, Kollagen-, Keratin-, Milcheiweiß-, Sojaprotein-, Mandelprotein- und Weizenproteinhydrolysate, deren Kondensationsprodukte mit Fettsäuren sowie quaternisierte Proteinhydrolysate. Besonders bevorzugt sind stark abgebaute Keratinhydrolysate mit Molmassen im Bereich von 400 bis 800. Ferner sind quaternierte Proteinhydrolysate, wie sie beispielsweise unter den Handelsbezeichnungen Gluadin® WQ (INCI-Bezeichnung: Laurdimonium Hydroxypropyl Hydrolyzed Wheat Protein) und Crotein® Q (INCI-Bezeichnung: Hydroxypropyltrimonium Hydrolyzed Collagen) vertrieben werden, erfindungsgemäß besonders bevorzugt.

Neben den quaternierten Proteinhydrolysaten stellen auch quaternäre Polymere erfindungsgemäß bevorzugte strukturverbessernde Verbindungen dar. Besonders bevorzugt sind die Polymere, die unter den Handelsbezeichnungen Mirapol® A15 (INCI-Bezeichnung: Polyquaternium-2), Onamer® M (INCI-Bezeichnung: Polyquaternium-1) und Merquat® 100 (INCI-Bezeichnung: Polyquaternium-6) vertrieben werden.

Ebenfalls faserstrukturverbessernde Wirkstoffe sind Mono-, Di- und Oligosaccharide wie beispielsweise Glucose, Galactose, Fructose, Fruchtzucker, Saccharose und Lactose. Weiterhin können auch Derivate dieser Pentosen und Hexosen, wie die entsprechenden On- und Uronsäuren (Zuckersäuren), Zuckeralkohole, Zuckeramine, wie beispielsweise N-Glucosamin, und Glykoside, erfindungsgemäß eingesetzt werden. Die Zuckersäuren können erfindungsgemäß in freier Form, in Form ihrer Salze, bevorzugt sind Calcium-, Magnesium- und Zink-Salze, und in Form ihrer Ester oder Lactone eingesetzt werden. Bevorzugte Zuckersäuren sind die Gluconsäure, Gluconsäure-γ-lacton, Lactobionsäure, die Glucuronsäure und ihre Mono- beziehungsweise Dilactone, die Pangaminsäure, die Zuckersäure, die Mannozuckersäure und ihre Mono- beziehungsweise Dilactone sowie die Schleimsäure und ihre Mono- beziehungsweise Dilactone. Bevorzugte Zuckeralkohole sind Sorbit, Mannit und Dulcit. Bevorzugte Glykoside sind die Methylglucoside. Glucose, N-Glucosamin und Gluconsäure sind aus dieser Gruppe besonders bevorzugt.

Auch gewisse Aminosäuren sind in Rahmen der vorliegenden Erfindung als haarstrukturverbessernde Wirkstoffe einsetzbar. Beispiele sind die in der DE-195 22 569, auf die hier ausdrücklich Bezug genommen wird, beschriebenen Aminosäuren Serin, Threonin und Tyrosin. Ferner sind auch Derivate des Serins, wie beispielsweise das Serinphosphat, erfindungsgemäß bevorzugt. Eine weitere snukturverbessernde Aminosäure stellt Lysin dar. Serin ist ein besonders bevorzugter faserstrukturverbessernder Wirkstoff.

Ebenfalls zur Strukturverbesserung können bestimmte Säuren, insbesondere α-Hydroxycarbonsäuren, und ihre Salze eingesetzt werden. Erfindungsgemäß bevorzugte strukturverbessernde Säuren sind Milchsäure, Äpfelsäure, Weinsäure, Glycerinsäure und Maleinsäure. Milchsäure ist besonders bevorzugt. Weiterhin verbessern spezielle Phosphonsäuren und ihre Salze die Struktur keratinischer Fasern. Erfindungsgemäß bevorzugte Phosphonsäuren sind die n-Octylphosphonsäure und die n-Decylphosphonsäure.

Weiterhin sind lipidlösliche Esteralkohole oder Esterpolyole für ihre strukturverbessernde Wirkung bekannt. Als lipidlöslich sind sie dann anzusehen, wenn sich 5 Gew.-% dieser Produkte in Cetylalkohol bei 80° C klar auflösen.

Die erfindungsgemäß geeigneten Esteralkohole oder Esterpolyole sind erhältlich durch Umsetzung eines Epoxyfettsäureesters mit Wasser oder ein- oder mehrwertigen Alkoholen mit 1 - 10 C-Atomen unter Öffnung des Epoxidrings und Ausbildung einer vizinalen Dihydroxyethyl- oder Hydroxy-alkoxy-ethylgruppe. Der Epoxyfettsäureester kann dabei auch ein Epoxidationsprodukt aus einem technischen Fettsäureester mit Anteilen gesättigter Fettsäuren sein. Der Epoxidsauerstoffgehalt sollte aber wenigstens 3 Gew.-%, bevorzugt 5 - 10 Gew.-%, betragen.

Die Epoxyfettsäureester sind dabei entweder epoxidierte Fettsäureester einwertiger Alkohole, also z.B. epoxidierter Ölsäuremethylester, Linolsäuremethylester, Ricinolsäuremethylester oder epoxidierte Fettsäureester mehrwertiger Alkohole, z.B. Glycerinmonooleat oder Propylenglycol-monooleat oder epoxidierte Fettsäuretriglyceride, z.B. Ölsäuretriglycerid oder ungesättigte Öle wie z.B. Olivenöl, Sojaöl, Sonnenblumenöl, Leinöl, Rüböl.

Technisch besonders interessant sind vor allem ungesättigte Fettsäuremethylester-Epoxide aus ungesättigten Pflanzenfettsäuren. So ist als Esterpolyol das Umsetzungsprodukt eines Pflanzenölfettsäuremethylester-Epoxidats mit einem Polyol mit 2 - 6 C-Atomen und 2 - 6 Hydroxylgruppen besonders bevorzugt. Als Polyole können dabei z.B. Ethylenglycol, 1,2-Propylenglycol, 1,3-Propylenglycol, Butandiol, Pentandiol, Hexandiol, Glycerin, Trimethylolpropan, Pentaerythrit, Sorbit oder Diglycerin enthalten sein.

Besonders gut eignet sich dabei für die erfindungsgemäßen Haarbehandlungsmittel als Esterpolyol das Umsetzungsprodukt eines Pflanzenfettsäuremethylester-Epoxidats mit Trimethylpropan und mit einer Hydroxylzahl von 350 - 450. Ein solches Produkt auf Basis von Sojaölfettsäuremethylester-Epoxid und Trimethylolpropan ist unter der Handelsbezeichnung Sovermol® 760 erhältlich.

Weiterhin kann als strukturverbessernder Wirkstoff Vitamin B₃ eingesetzt werden. Unter dieser Bezeichnung werden häufig die Verbindungen Nicotinsäure und Nicotinsäureamid (Niacinamid) geführt. Erfindungsgemäß bevorzugt ist das Nicotinsäureamid.

Auch Vitamin H ist als strukturverbessernder Wirkstoff im Sinne der vorliegenden Erfindung einsetzbar. Als Vitamin H wird die Verbindung (3a*S*,4*S*, 6a*R*)-2-Oxohexahydrothienol[3,4-*d*]-imidazol-4-valeriansäure bezeichnet, für die sich aber zwischenzeitlich der Trivialname Biotin durchgesetzt hat.

Die erfindungsgemäßen Mitttel enthalten die strukturverbessernden Wirkstoffe bevorzugt in Mengen von 0,1 bis 5 Gew.-%, besonders bevorzugt in Mengen von 0,2 bis 2 Gew.-%.

In einer bevorzugten Ausführungsform der vorliegenden Erfindung enthalten die Mittel weiterhin eine Magnesiumverbindung. Die erfindungsgemäßen Mittel können durch Zugabe der Mg²⁺-Kationen hinsichtlich ihre strukturerhaltenden Eigenschaften weiter optimiert werden. Bevorzugte Magnesiumverbindungen sind anorganische und organische Mg²⁺-Salze, wie beispielsweise die Halogenide, die Carbonate und Hydrogencarbonate, das Acetat und das Citrat.

In einer weiteren bevorzugten Ausführungsform dieses Gegenstandes der Erfindung enthalten die Mittel mindestens ein Farbstoffvorprodukt vom Typ der Entwickler enthält.

Hinsichtlich der bevorzugten Entwicklerkomponenten sei an dieser Stelle explizit auf die obigen Ausführungen verwiesen.

Mittel zur Farbveränderung keratinischer Fasern, das unmittelbar vor der Anwendung durch Mischen einer ersten Komponente, enthaltend mindestens eine Entwicklerkomponente, mit einer zweiten Komponente, enthaltend mindestens ein Oxidationsmittel, erhalten wird, dadurch gekennzeichnet, daß es mindestens 0,1 Gew.-%, bezogen auf die Anwendungszubereitung, einer gegebenenfalls hydratisierten SiO₂-Verbindung in Form von Kieselgelen (Silicagel) oder als Wasserglas und mindestens einen strukturverbessernden Wirkstoff, der ausgewählt ist aus Panthenol, physiologisch verträglichen Panthenol-Derivaten, Mono-, Di- und Oligosacchariden, Serin, Niacinamid, Polyvinylpyrrolidon, Gluconsäure, Biotin und den lipidlöslichen Esteralkoholen oder Esterpolyolen, enthält.

Besonders bevorzugt ist der Einsatz von mindestens 0,25 Gew.-%, insbesondere mindestens 0,3 Gew.-%, jeweils bezogen auf die Anwendungszubereitung, einer gegebenenfalls hydratisierten SiO₂-Verbindung.

Weiterhin können die Mittel der verschiedenen Gegenstände dieser Erfindung zur Nuancierung mindestens eine Kupplerkomponente enthalten. Auch an dieser Stelle wird explizit auf die obigen Ausführungen verwiesen.

Die erfindungsgemäßen Mittel können weiterhin alle für solche Zubereitungen bekannten Wirk-, Zusatz- und Hilfsstoffe enthalten. In vielen Fällen enthalten die Färbemittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslichmachende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- lineare Fettsäuren mit 10 bis 22 C-Atomen (Seifen),
- Ethercarbonsäuren der Formel R-O-(CH₂-CH₂O)ₓ -CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)ₓ-SO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C₈-C₂₂-Carbonsäuren, wie Ölsäure, Stearinsäure, Isostearinsäure und Palmitinsäure.

Als zwitterionische Tenside werden solche oberflächenaktiven Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Unter ampholytischen Tensiden werden solche oberflächenaktiven Verbindungen verstanden, die außer einer C₈₋₁₈-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Nichtionische Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂₋₂₂-Fettsäuremono- und -diester von Anlagerungsprodukten von 1 bis 30 Mol Ethylenoxid an Glycerin,
- C₈₋₂₂-Alkylmono- und -oligoglycoside und deren ethoxylierte Analoga,
- Anlagerungsprodukte von 5 bis 60 Mol Ethylenoxid an Rizinusöl und gehärtetes Rizinusöl,
- Anlagerungeprodukte von Ethylenoxid an Sorbitanfettsäureester
- Anlagerungsprodukte von Ethylenoxid an Fettsäurealkanolamide.

Beispiele für die in den erfindungsgemäßen Haarbehandlungsmitteln verwendbaren kationischen Tenside sind insbesondere quartäre Ammoniumverbindungen. Bevorzugt sind Ammoniumhalogenide wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere erfindungsgemäß verwendbare kationische Tenside stellen die quaternisierten Proteinhydrolysate dar.

Erfindungsgemäß ebenfalls geeignet sind kationische Silikonöle wie beispielsweise die im Handel erhältlichen Produkte Q2-7224 (Hersteller: Dow Corning; ein stabilisiertes Trimethylsilylamodimethicon), Dow Corning 929 Emulsion (enthaltend ein hydroxylamino-modifiziertes Silicon, das auch als Amodimethicone bezeichnet wird), SM-2059 (Hersteller: General Electric), SLM-55067 (Hersteller: Wacker) sowie Abil® -Quat 3270 und 3272 (Hersteller: Th. Goldschmidt; diquaternäre Polydimethylsiloxane, Quaternium-80).

Alkylamidoamine, insbesondere Fettsäureamidoamine wie das unter der Bezeichnung Tego Amid® S 18 erhältliche Stearylamidopropyldimethylamin, zeichnen sich neben einer guten konditionierenden Wirkung speziell durch ihre gute biologische Abbaubarkeit aus. Ebenfalls sehr gut biologisch abbaubar sind quaternäre Esterverbindungen, sogenannte "Esterquats", wie die unter dem Warenzeichen Stepantex® vertriebenen Methylhydroxyalkyldialkoyloxyalkyl-ammoniummethosulfate sowie die unter dem Warenzeichen Dehyquart® vertriebenen Produkte wie Dehyquart® AU-46.

Ein Beispiel für ein als kationisches Tensid einsetzbares quaternäres Zuckerderivat stellt das Handelsprodukt Glucquat® 100 dar, gemäß INCI-Nomenklatur ein "Lauryl Methyl Gluceth-10 Hydroxypropyl Dimonium Chloride".

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Weiterhin können die erfindungsgemäßen Haarbehandlungsmittel bevorzugt noch einen konditionierenden Wirkstoff, ausgewählt aus der Gruppe, die von kationischen Tensiden, kationischen Polymeren, Alkylamidoaminen, Paraffinölen, pflanzlichen Ölen und synthetischen Ölen gebildet wird, enthalten.

Als konditionierende Wirkstoffe bevorzugt sein können kationische Polymere. Dies sind in der Regel Polymere, die ein quartäres Stickstoffatom, beispielsweise in Form einer Ammoniumgruppe, enthalten.
Bevorzugte kationische Polymere sind beispielsweise
- quaternisierte Cellulose-Derivate, wie sie unter den Bezeichnungen Celquat® und Polymer JR® im Handel erhältlich sind. Die Verbindungen Celquat® H 100, Celquat® L 200 und Polymer JR® 400 sind bevorzugte quaternierte Cellulose-Derivate.
- polymere Dimethyldiallylammoniumsalze und deren Copolymere mit Acrylsäure sowie Estern und Amiden von Acrylsäure und Methacrylsäure. Die unter den Bezeichnungen Merquat® 100 (Poly(dimethyldiallylammoniumchlorid)), Merquat® 550 (Dimethyldiallylammoniumchlorid-Acrylamid-Copolymer) und Merquat® 280 (Dimethyldiallylammoniumchlorid-Acrylsäure-Copolymer im Handel erhältlichen Produkte sind Beispiele für solche kationischen Polymere.
- Copolymere des Vinylpyrrolidons mit quaternierten Derivaten des Dialkylaminoacrylats und -methacrylats, wie beispielsweise mit Diethylsulfat quaternierte Vinylpyrrolidon-Dimethylaminomethacrylat-Copolymere. Solche Verbindungen sind unter den Bezeichnungen Gafquat® 734 und Gafquat®755 im Handel erhältlich.
- Vinylpyrrolidon-Methoimidazoliniumchlorid-Copolymere, wie sie unter der Bezeichnung Luviquat® angeboten werden.
- quaternierter Polyvinylalkohol
sowie die unter den Bezeichnungen
- Polyquatemium 2,
- Polyquatemium 17,
- Polyquaternium 18 und
- Polyquaternium 27 bekannten Polymeren mit quartären Stickstoffatomen in der Polymerhauptkette.

Besonders bevorzugt sind kationische Polymere der vier erstgenannten Gruppen, ganz besonders bevorzugt sind Polyquaternium-2, Polyquaternium-10 und Palyquaternium-22.

Als konditionierende Wirkstoffe weiterhin geeignet sind Silikonöle, insbesondere Dialkylund Alkylarylsiloxane, wie beispielsweise Dimethylpolysiloxan und Methylphenylpolysiloxan, sowie deren alkoxylierte und quaternierte Analoga. Beispiele für solche Silikone sind die von Dow Corning unter den Bezeichnungen DC 190, DC 200, DC 344, DC 345 und DC 1401 vertriebenen Produkte sowie die Handelsprodukte Q2-7224 (Hersteller: Dow Coming; ein stabilisiertes Trimethylsilylamodimethicon), Dow Corning® 929 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abil® -Quat 3270 und 3272 (Hersteller: Th. Goldschmidt; diquatemäre Polydimethylsiloxane, Quaternium-80).

Ebenfalls einsetzbar als konditionierende Wirkstoffe sind Paraffinöle, synthetisch hergestellte oligomere Alkene sowie pflanzliche Öle wie Jojobaöl, Sonnenblumenöl, Orangenöl, Mandelöl, Weizenkeimöl und Pfirsichkernöl.

Gleichfalls geeignete haarkonditionierende Verbindungen sind Phospholipide, beispielsweise Sojalecithin, Ei-Lecithin und Kephaline.

Weitere Wirk-, Hilfs- und Zusatzstoffe sind beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- zwitterionische und amphotere Polymere wie beispielsweise Acrylamidopropyl-trimethylammoniumchlorid/Acrylat-Copolymere und Octylacrylamid/Methyl-methacrylat/tert-Butylaminoethylmethacrylat/2-Hydroxypropylmethacrylat-Copolymere,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Maleinsäureanhydrid-Copolymere und Acrylsäure/Ethylacrylat/N-tert.Butyl-acrylamid-Terpolymere,
- Verdickungsmittel wie Agar-Agar, Guar-Gum, Alginate, Xanthan-Gum, Gummi arabicum, Karaya-Gummi, Johannisbrotkemmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol,
- Strukturanten wie Maleinsäure und Milchsäure,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösungsmittel und -vermittler wie Ethanol, Isopropanol, Ethylenglykol, Propylenglykol, Glycerin und Diethylenglykol,
- quaternierte Amine wie Methyl-1-alkylamidoethyl-2-alkylimidazolinium-methosulfat
- Entschäumer wie Silikone,
- Farbstoffe zum Anfärben des Mittels,
- Antischuppenwirkstoffe wie Piroctone Olamine, Zink Omadine und Climbazol,
- Lichtschutzmittel, insbesondere derivatisierte Benzophenone, Zimtsäure-Derivate und Triazine,
- Substanzen zur Einstellung des pH-Wertes, wie beispielsweise übliche Säuren, insbesondere Genußsäuren und Basen,
- Wirkstoffe wie Allantoin, Pyrrolidoncarbonsäuren und deren Salze sowie Bisabolol,
- Vitamine, Provitamine und Vitaminvorstufen, insbesondere solche der Gruppen A, B₃, B₅, B₆, C, E, F und H,
- Cholesterin,
- Konsistenzgeber wie Zuckerester, Polyolester oder Polyolalkylether,
- Fette und Wachse wie Walrat, Bienenwachs, Montanwachs und Paraffine,
- Fettsäurealkanolamide,
- Komplexbildner wie EDTA, NTA, β-Alanindiessigsäure und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex, Styrol/PVP- und Styrol/Acrylamid-Copolymere
- Perlglanzmittel wie Ethylenglykolmono- und -distearat sowie PEG-3-distearat,
- Pigmente,
- Stabilisierungsmittel für Wassserstoffperoxid und andere Oxidationsmittel,
- Treibmittel wie Propan-Butan-Gemische, N₂O, Dimethylether, CO₂ und Luft,
- Antioxidantien.

Bezüglich weiterer fakultativer Komponenten sowie die eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.

Ein dritter Gegenstand der vorliegenden Erfindung ist ein Verfahren zur Blondierung keratinischer Faserm, bei dem die Fasern mit einem der oben beschriebenen Mittel behandelt werden.

Ein vierter Gegenstand der vorliegenden Erfindung ist ein Verfahren zur oxidativen Färbung keratinischer Fasern, bei dem die Fasern mit einem der oben beschriebenen Mittel behandelt werden.

Die folgenden Beispiele sollen den Gegenstand der vorliegenden Erfindung erläutern ohne ihn jedoch zu beschränken.

### Beispiele

Sämtliche Mengenangaben verstehen sich in Gewichtsprozent sofern nichts anderes vermerkt ist. Der Grad der Haarschädigung wurde an Haarfasern der Fa. Alkinco (Code 6634, natural dark brown) durch die Bestimmung der elastischen Daten im Hook'schen Bereich (1% Dehnung) der Fasern ermittelt.

### Versuchsreihe 1: Blondierung

Nach der Ermittlung des Haarquerschnitts im nassen Zustand wurden mit dem Vollautomat MTT 600 der Fa. Dia Stron die Meßwerte für 1% Dehnung der Fasern im nassen Zustand ermittelt. Anschließend wurden die Fasern getrocknet und jeweils für 30 Minuten mit einem Blondiermittel der jeweiligen Prüfrezeptur behandelt. Die Prüfrezeptur wurde dabei unmittelbar vor der Anwendung durch Mischung von 50g der Rezeptur A, B oder C jeweils mit 50g der Entwicklerlösung und 12,5 g des Blondaktivators hergestellt. Nach einer gründlichen Spülung wurden die Fasern für 30 Minuten bei 35°C getrocknet. Die Blondierung wurde gegebenenfalls zwei weitere Male wiederholt. Anschließend wurden die Fasern erneut im nassem Zustand vermessen.

| Vergleichsrezeptur A | |
|---|---|
| Hydrenol® D ¹ | 10,00 |
| Lorol®, techn. ² | 2,00 |
| Texapon® NSO ³ | 26,50 |
| Ammoniumsulfat | 1,00 |
| Ammoniaklösung, 25%ig | 7,60 |
| Gluadin® W40 ⁴ | 0,35 |
| Wasser | ad 100 |
| pH | 10,7 |

| | |
|---|---|
| ¹ C₁₆₋₁₈-Fettalkohol (INCI-Bezeichnung: Cetearyl alcohol) (HENKEL) | |
| ² C₁₂₋₁₈-Fettalkohol (INCI-Bezeichnung: Coconut alcohol) (HENKEL) | |
| ³ Laurylethersulfat, Natriumsalz (ca. 27,5% Aktivsubstanz; INCI-Bezeichnung: Sodium Laureth Sulfate) (HENKEL) | |
| ⁴ Weizenproteinhydrolysat (ca. 40% Aktivsubstanz; INCI-Bezeichnung: Aqua (Water), Hydrolyzed Wheat Protein, Sodium Benzoate, Phenoxyethanol, Methylparaben, Propylparaben) (HENKEL) | |

| Erfindungsgemäße Rezeptur B | |
|---|---|
| Hydrenol® D | 10,00 |
| Lorol®, techn. | 2,00 |
| Texapon® NSO | 26,50 |
| Wasserglas 40/42 ⁵ | 0,50 |
| Ammoniumsulfat | 1,00 |
| Ammoniaklösung, 25%ig | 7,60 |
| Gluadin® W40 | 0,35 |
| Wasser | ad 100 |
| pH | 10,7 |

| | |
|---|---|
| ⁵ ca. 40% Aktivsubstanz (INCI-Bezeichnung: Sodium Silicate) (HENKEL) | |

| Erfindungsgemäße Rezeptur C | |
|---|---|
| Hydrenol® D | 10,00 |
| Lorol®, techn. | 2,00 |
| Texapon® NSO | 26,50 |
| Wasserglas 40/42 | 2,50 |
| Ammoniumsulfat | 1,00 |
| Ammoniaklösung, 25%ig | 7,60 |
| Gluadin® W40 | 0,35 |
| Wasser | ad 100 |
| pH | 10,7 |

| Entwicklerlösung | |
|---|---|
| Dipicolinsäure | 0,10 |
| Ammoniaklösung, 25%ig | 0,70 |
| Natriumpyrophosphat, sauer | 0,03 |
| Turpinal® SL ⁶ | 1,50 |
| Texapon® N25 ⁷ | 2,00 |
| Dow Corning DB-110A ⁸ | 0,07 |
| Acrysol® 33 ⁹ | 12,00 |
| H₂O₂-Lösung, 51,5%ig | 22,40 |
| Wasser | ad 100 |
| pH | 3,8 |

| | |
|---|---|
| ⁶ 1-Hydroxyethan-1,1-diphosphonsäure (ca. 60% Aktivsubstanz; INCI-Bezeichnung: Etidronic Acid) (Cognis) | |
| ⁷ Natriumlaurylethersulfat (ca. 28% Aktivsubstanz; INCI-Bezeichnung: Sodium Laureth Sulfate) (Cognis) | |
| ⁸ nichtionogene Siliconemulsion (ca. 10% Aktivsubstanz; INCI-Bezeichnung: Dimethicone) (Dow Corning) | |
| ⁹ wässrige Acrylatdispersion (ca. 28% Aktivsubstanz; INCI-Bezeichnung: Acrylates Copolymer) (Rohm & Haas) | |

| Blondaktivator (kommerziell von Degussa erhältlich) | |
|---|---|
| Ammoniumperoxidisulfat | 96,0 |
| Aerosil® 200 ¹⁰ | 4,0 |

| | |
|---|---|
| ¹⁰ Siliciumdioxid (INCI-Bezeichnung: Silica) (Degussa Hüls) | |

Die in Tabelle 1 angegeben Absolutwerte sind jeweils die Differenzen der Meßwerte vor und nach der Blondierung. Je kleiner die Differenzen sind, umso weniger haben sich die Eigenschaften der Fasern verändert und umso weniger stark geschädigt sind die Fasern. Negative Differenzen bedeuten, daß die Fasern nach der Behandlung bessere mechanische Eigenschaften aufweisen als vor der Behandlung.
Die prozentualen Werte sind das Ergebnis eines statistischen, zweiseitigen t-Tests zwischen den Werten der erfindungsgemäßen Rezepturen mit Wasserglas und der Vergleichsrezeptur. Werte oberhalb von 95% zeigen einen signifikanten Unterschied der Meßwerte an. Werte zwischen 90 und 95% sind als deutliche Tendenz zu werten. Die statistische Auswertung wurde anhand der Meßergebnisse von 40 Fasern ausgeführt.

**Tabelle I**

| | Einmalige Behandlung | | | Dreifache Behandlung | | |
|---|---|---|---|---|---|---|
| | Spannungswert bei 1% Dehnung [10⁻⁶* N/µm²] | Elastizitätsmodul [10⁸* N/mm] | Arbeit bei 1% Dehnung [10⁻⁶*J] | Spannungswert bei 1% Dehnung [10⁻⁶* N/µm²] | Elastizitätsmodul [10⁸* N/mm] | Arbeit bei 1% Dehnung [10⁻⁶*J] |
| Vergleichsrezeptur A | 2,35 | 2,63 | 1,29 | 7,25 | 4,79 | 5,37 |
| Rezeptur B (0,09 Gew.-% Wasserglas) | 0,04 (98,80%) | 0,15 (98,72%) | -0,15 (98,71%) | 6,30 (59,72%) | 4,05 (47,20%) | 4,97 (46,11%) |
| Rezeptur C (0,44 Gew.-% Wasserglas) | 0,42 (94,26%) | 0,38 (95,78%) | 0,11 (91,36%) | 4,72 (98,16%) | 1,98 (98,89%) | 3,91 (98,91%) |

Die statistische Auswertung zeigt deutlich, daß ein Zusatz von 0,09 Gew.-% Wasserglas bei einer einmaligen Blondierung zu einer geringeren Haarschädigung führt. Der Einsatz von 0,44 Gew.-% Wasserglas hat sogar bei einer dreimaligen Blondierung einen positiven Einfluß auf die Haarschädigung (Verringerung der Haarschädigung).

### Versuchsreihe 2: Oxidative Färbung

Nach der Ermittlung des Haarquerschnitts im nassen Zustand wurden mit dem Vollautomat MTT 600 der Fa. Dia Stron die Meßwerte für 1 % Dehnung der Fasern im nassen Zustand ermittelt. Anschließend wurden die Fasern getrocknet und jeweils für 30 Minuten mit einem Färbemittel der jeweiligen Prüfrezeptur behandelt. Die Prüfrezeptur wurde dabei unmittelbar vor der Anwendung durch Mischung der Rezeptur D beziehungsweise E mit der Entwickleremulsion im Verhältnis 2:1 erhalten. Nach einer gründlichen Spülung wurden die Fasern für 30 Minuten bei 35°C getrocknet. Die Färbung wurde gegebenenfalls zwei weitere Male wiederholt. Anschließend wurden die Fasern erneut im nassem Zustand vermessen.

| Vergleichsrezeptur D | |
|---|---|
| Stenol® 1618 ¹¹ | 6,00 |
| Lorol®, techn. | 2,00 |
| Eumulgin® B2 ¹² | 2,0 |
| p-Toluylendiamin | 0,27 |
| p-Aminophenol | 0,01 |
| Resorcin | 0,02 |
| 4-Chlorresorcin | 0,03 |
| 2-Methylamino-3-amino-6-methoxypyridin | 0,03 |
| Turpinal® SL | 0,2 |
| Ammoniumsulfat | 0,9 |
| Ascorbinsäure | 0,5 |
| Natriumsulfit | 0,5 |
| Ammoniaklösung, 25%ig | 7,25 |
| Parfüm | 0,2 |
| Wasser | ad 100 |

| | |
|---|---|
| ¹¹ C₁₆₋₁₈-Fettalkohol (INCI-Bezeichnung: Cetearyl Alcohol) (Cognis) | |
| ¹² Cetylstearylalkohol mit ca. 20-EO-Einheiten (INCI-Bezeichnung: Ceteareth-20) (Cognis) | |

| Erfindungsgemäße Rezeptur E | |
|---|---|
| Stenol® 1618 | 6,00 |
| Lorol®, techn. | 2,00 |
| Eumulgin® B2 | 2,0 |
| p-Toluylendiamin | 0,27 |
| p-Aminophenol | 0,01 |
| Resorcin | 0,02 |
| 4-Chlorresorcin | 0,03 |
| 2-Methylamino-3-amino-6-methoxypyridin | 0,03 |
| Turpinal® SL | 0,2 |
| Wasserglas 40/42 | 0,5 |
| Ammoniumsulfat | 0,9 |
| Ascorbinsäure | 0,5 |
| Natriumsulfit | 0,5 |
| Ammoniaklösung, 25%ig | 7,25 |
| Wasser | ad 100 |

| Entwickleremulsion | |
|---|---|
| Lorol® C16 ¹³ | 3,5 |
| Eumulgin® B2 | 1,0 |
| Disponil® FES 77IS ¹⁴ | 2,5 |
| Ammoniak-Lösung, 25%ig | 0,65 |
| Dipicolinsäure | 0,1 |
| Natriumpyrophosphat | 0,03 |
| Turpinal® SL | 1,5 |
| Wasserstoffperoxidlösung, 50%ig | 12,0 |
| Acrysol® 33 | 10,0 |

| | |
|---|---|
| ¹³ Cetylalkohol (INCI-Bezeichnung: Cetyl Alcohol) | |
| ¹⁴ ethoxyliertes Fettalkoholsulfat (INCI-Bezeichnung: Sodium Coceth-30 Sulfate) (Cognis) | |

Die in Tabelle II angegeben Absolutwerte sind jeweils die Differenzen der Meßwerte vor und nach der Färbung. Je kleiner die Differenzen sind, umso weniger haben sich die Eigenschaften der Fasern verändert und umso weniger stark geschädigt sind die Fasern. Die prozentualen Werte sind das Ergebnis eines statistischen, zweiseitigen t-Tests zwischen den Werten der Rezepturen mit Wasserglas und der Vergleichsrezeptur. Werte oberhalb von 95% zeigen einen signifikanten Unterschied der Meßwerte an. Werte zwischen 90 und 95% sind als deutliche Tendenz zu werten. Die statistische Auswertung wurde anhand der Meßergebnisse von 40 Fasern ausgeführt.

**Tabelle II**

| | Einmalige Behandlung | | | Dreifache Behandlung | | |
|---|---|---|---|---|---|---|
| | Spannungswert bei 1% Dehnung [10⁻⁶* N/µm²] | Elastizitätsmodul [10⁸* N/mm] | Arbeit bei 1% Dehnung [10⁻⁶*J] | Spannungswert bei 1% Dehnung [10⁻⁶* N/µm²] | Elastizitätsmodul [10⁸* N/mm] | Arbeit bei 1% Dehnung [10⁻⁶*J] |
| Vergleichsrezeptur D | 1,76 | 1,96 | 1,14 | 3,26 | 3,14 | 1,97 |
| Rezeptur E mit 0,13 Gew.-% Wasserglas | 0,00 (97,75%) | -0,18 (99,17%) | -0,32 (99,22%) | 1,2 (98,52%) | 1,10 (97,79%) | 0,37 (99,59%) |

Die statistische Auswertung zeigt deutlich, daß schon ein Zusatz von 0,13 Gew.-% Wasserglas bei bis zu dreimaliger Färbung zu einer geringeren Haarschädigung führt.

| *Versuchsreihe 3: Oxidative Färbungen* Färbecremes: | | | | | |
|---|---|---|---|---|---|
| Rohstoff/Rezeptur-Nr. | 3.1 | 3.2 | 3.3 | 3.4 | 3.5 |
| C₁₂₋₁₈ Fettalkohol | 1,2 | 1,2 | 1,2 | 1,2 | 1,2 |
| C₁₆₋₁₈ Fettalkohol | 4,0 | 4,0 | 4,0 | 4,0 | 4,0 |
| Foryl® 100¹⁵ | 0,8 | 0,8 | 0,8 | 0,8 | 0,8 |
| Isostearinsäure | 2,0 | 2,0 | 2,0 | 2,0 | 2,0 |
| Lamesoft® PO 65¹⁶ | 4,0 | 4,0 | 4,0 | 4,0 | 4,0 |
| Cetiol ®OE¹⁷ | 0,5 | 0,5 | 0,5 | 0,5 | 0,5 |
| Natriumdisulfit | 0,3 | 0,3 | 0,3 | 0,3 | 0,3 |
| Turpinal® SL | 0,2 | 0,2 | 0,2 | 0,2 | 0,2 |
| Ammoniumsulfat | 0,5 | 0,5 | 0,5 | 0,5 | 0,5 |
| p-Aminophenol | 0,35 | 0,35 | 0,35 | 0,35 | 0,35 |
| p-Toluylendiaminsulfat | 0,85 | 0,85 | 0,85 | 0,85 | 0,85 |
| 2-Methylresorcin | 0,14 | 0,14 | 0,14 | 0,14 | 0,14 |
| 5-Amino-2-methylphenol | 0,42 | 0,42 | 0,42 | 0,42 | 0,42 |
| Wasserglas 40/42 | 0,6 | 0,6 | 0,6 | 0,6 | 0,6 |
| Pyridoxin | 0,1 | -- | -- | -- | -- |
| Nicotinsäureamid | 0,2 | -- | -- | -- | -- |
| Mirapol® A15¹⁸ | -- | 1,0 | -- | -- | -- |
| Gluadin® WQ¹⁹ | -- | 1,0 | -- | -- | -- |
| Merquat® 100²⁰ | -- | -- | 1,0 | -- | -- |
| Dowicil® 200²¹ | -- | -- | 1,0 | -- | -- |
| Äpfelsäure | -- | -- | -- | 0,5 | -- |
| Milchsäure | -- | -- | -- | 0,5 | -- |
| Lysin | -- | -- | -- | -- | 1,0 |
| Promois® WK²² | -- | -- | -- | -- | 1,0 |
| Ammoniak, 25%ig | ad pH 10 | ad pH 10 | ad pH 10 | ad pH 10 | ad pH 10 |
| Wasser | ad 100 | ad 100 | ad 100 | ad 100 | ad 100 |

| | | | | | |
|---|---|---|---|---|---|
| ¹⁵ C₁₂₋₁₈-Alkohol mit ca. 9 EO-Einheiten (INCI-Bezeichnung: Laureth-10) (Cognis) | | | | | |
| ¹⁶ Alkylpolyglucosid-Ölsäuremonoglycerid-Gemisch (INCI-Bezeichnung: Coco-Glucoside, Glyceryl Oleate, Aqua (Water)) (Cognis) | | | | | |
| ¹⁷ Dioctylether (INCI-Bezeichnung: Dicaprylyl Ether) (Cognis) | | | | | |
| ¹⁸ quaternäres Polymer (ca. 64% Aktivsubstanz; INCI-Bezeichnung: Polyquaternium-2) (Miranol) | | | | | |
| ¹⁹ quaterniertes Weizenproteinhydrolysat (ca. 33% Aktivsubstanz; INCI-Bezeichnung: Laurdimonium Hydroxypropyl Hydrolyzed Wheat Protein) (Cognis) | | | | | |
| ²⁰ Poly(dimethyldiallylammoniumchlorid) (ca. 40% Aktivsubstanz; INCI-Bezeichnung: Polyquaternium-6) (Chemviron) | | | | | |
| ²¹ cis-1(3-Chlorallyl)-3,5,7-triaza-1-azoniaadamantanchlorid (INCI-Bezeichnung: Quaternium-15) (Cognis) | | | | | |
| ²² Keratinhydrolysat (INCI-Bezeichnung: Hydrolyzed Keratin) (Interorgana) | | | | | |

| Oxidationsmittelzubereitungen: | | | | | |
|---|---|---|---|---|---|
| Rohstoff/Rezeptur-Nr. | 3.1 | 3.2 | 3.3 | 3.4 | 3.5 |
| Texapon NSO | 2,1 | 2,1 | 2,1 | 2,1 | 2,1 |
| C₁₂-₁₈ Fettalkohol | 3,0 | 3,0 | 3,0 | 3,0 | 3,0 |
| Wasserstoffperoxid, 50 %ig | 12,0 | 12,0 | 12,0 | 12,0 | 12,0 |
| Turpinal® SL | 1,5 | 1,5 | 1,5 | 1,5 | 1,5 |
| EDTA, Dinatriumsalz | 0,3 | 0,3 | 0,3 | 0,3 | 0,3 |
| Acrysol® 33 | 3,0 | 3,0 | 3,0 | 3,0 | 3,0 |
| n-Decylphosphonsäure | 2,0 | 2,0 | 2,0 | 2,0 | 2,0 |
| Ca-Gluconat | 1,0 | -- | -- | -- | -- |
| Glycerinsäure | -- | 1,0 | -- | -- | -- |
| Pangaminsäure | -- | -- | 0,5 | -- | -- |
| Lactobionsäure | -- | -- | 0,5 | -- | -- |
| Serin | -- | -- | -- | 1,0 | -- |
| Panthenol | -- | -- | -- | 1,0 | -- |
| Onamer® M²³ | -- | -- | -- | -- | 2,0 |
| Crotein® Q²⁴ | -- | -- | -- | -- | 1,0 |
| Ammoniak, 25%ig | ad pH 4,0 | ad pH 4,0 | ad pH 4,0 | ad pH 4,0 | ad pH 4,0 |
| Wasser | ad 100 | ad 100 | ad 100 | ad 100 | ad 100 |

| | | | | | |
|---|---|---|---|---|---|
| ²³ poly(dimethylbutenylammoniumchlorid)-alpha,omega-bis-(triethanolammonium-chlorid) (ca. 30% Aktivsubstanz; INCI-Bezeichnung: Polyquaternium-1) (Onyx) | | | | | |
| ²⁴ Trimethylstearylammoniumproteinhydrolysat (ca. 90% Aktivsubstanz; INCI-Bezeichnung: Hydroxypropyltrimonium Hydrolyzed Collagen) (Croda) | | | | | |

Beide Komponenten (Färbecreme und Oxidationsmittelzubereitung) der jeweiligen Rezepturen 3.1 bis 3.5 wurden unmittelbar vor der Anwendung miteinander gemischt, auf Haarsträhnen von ca. 1 g Gewicht (Kerling naturweiß) im Gewichtsverhältnis 4:1 aufgetragen und dort für eine halbe Stunde belassen. Anschließend wurden die Fasern gespült, gewaschen und getrocknet. Parallel dazu wurden Haarsträhnen jeweils ohne die erfindungsgemäßen Wirkstoffkombinationen aus Wasserglas und den entsprechenden Strukturanten auf die gleiche Weise behandelt.

Ein Vergleich der Haarsträhnen ergab, daß die mit den erfindungsgemäßen Rezepturen behandelten Strähnen sich deutlich weicher und gepflegter anfühlten und sich wesentlich besser kämmen ließen.

| *Versuchsreihe 4: Dauerwellfixierung* | | | |
|---|---|---|---|
| Rohstoff/ Rezeptur-Nr. | 4.1 | 4.2 | 4.3 |
| Plantacare® 818 UP²⁵ | 5,0 | 5,0 | 5,0 |
| gehärtetes Rizinusöl | 2,0 | 2,0 | 2,0 |
| Lamesoft® PO 65 | 1,0 | 1,0 | 1,0 |
| Kaliumbromat | 3,5 | -- | -- |
| Wasserstoffperoxid | -- | 3,0 | 3,0 |
| Nitrilotriessigsäure | 0,3 | 0,3 | 0,3 |
| Phosphorsäure | 0,2 | 0,2 | 0,2 |
| Wasserglas 40/42 | 0,5 | 0,5 | 0,5 |
| Gluadin® WQ | 1,0 | -- | -- |
| Panthenol | 0,2 | -- | -- |
| Merquat® 100 | -- | 0,2 | -- |
| Mirapol® A 15 | -- | 0,2 | -- |
| Nicotinsäureamid | -- | -- | 0,1 |
| Pyridoxin | -- | -- | 0,1 |
| Wasser | ad 100 | ad 100 | ad 100 |

| | | | |
|---|---|---|---|
| ²⁵ C₈₋₁₄-Alkylpolyglucosid (ca. 52% Aktivsubstanz; INCI-Bezeichnung: Coco-Glucoside) (Cognis) | | | |

Die Fixierlösungen wurden in Vergleichsversuchen jeweils halbseitig mit und ohne die erfindungsgemäßen Kombinationen aus Wasserglas und den Strukturanten zur Fixierung von Fasern eingesetzt, die vorher mit dem Wellmittel einer kommerziellen Dauerwelle (Poly Style Schaumdauerwelle) behandelt wurden. In jedem Falle wurde ein besserer Griff und eine bessere Kämmbarkeit der Fasern festgestellt, die mit den erfindungsgemäßen Formulierungen behandelt worden waren.

## Patentansprüche

1. Verwendung von gegebenenfalls hydratisierten SiO₂-Verbindungen zur Verringerung der Schädigung keratinischer Fasern, insbesondere menschlicher Haare, durch oxidative Prozesse.

2. Verwendung nach Anspruch 1, **dadurch gekennzeichnet, daß** die gegebenenfalls hydratisierte SiO₂-Verbindung ein Silicagel ist.

3. Verwendung nach einem der Ansprüche 1, **dadurch gekennzeichnet, daß** die gegebenenfalls hydratisierte SiO₂-Verbindung eine wäßrigen Lösung eines Silikates der Formel (SiO₂)ₙ(Na₂O)ₘ(K₂O)ₚ ist, wobei n steht für eine positive rationale Zahl und m und p stehen unabhängig voneinander für eine positive rationale Zahl oder für 0, mit den Maßgaben, daß mindestens einer der Parameter m oder p von 0 verschieden ist und das Verhältnis zwischen n und der Summe aus m und p zwischen 1:4 und 4:1 liegt.

4. Verwendung nach einem der Ansprüche 1 bis 3, **dadurch gekennzeichnet, daß** der oxidative Prozeß eine Blondierung ist.

5. Verwendung nach einem der Ansprüche 1 bis 3, **dadurch gekennzeichnet, daß** der oxidative Prozeß eine oxidative Haarfärbung ist.

6. Mittel zur Behandlung keratinischer Fasern, enthaltend ein Oxidationsmittel, mindestens eine gegebenenfalls hydratisierte SiO₂-Verbindung in Form von Kieselgelen (Silicagel) oder als Wasserglas und mindestens einen strukturverbessernden Wirkstoff, der ausgewählt ist aus Panthenol, physiologisch verträglichen Panthenol-Derivaten, Mono-, Di- und Oligosacchariden, Serin, Niacinamid, Polyvinylpyrrolidon, Gluconsäure, Biotin und den lipidlöslichen Esteralkoholen oder Esterpolyolen.

7. Mittel nach Anspruch 6, **dadurch gekennzeichnet, daß** es mindestens 0,3 Gew.-% der gegebenenfalls hydratisierten SiO₂-Verbindung enthält.

8. Mittel nach einem der Ansprüche 6 oder 7, **dadurch gekennzeichnet, daß** es den strukturverbessernden Wirkstoff in Mengen von 0,1 bis 5 Gew.-% enthält.

9. Mittel nach einem der Ansprüche 6 bis 8, **dadurch gekennzeichnet, daß** es weiterhin eine Magnesiumverbindung enthält.

10. Mittel nach einem der Ansprüche 6 bis 9, **dadurch gekennzeichnet, daß** es mindestens ein Farbstoffvorprodukt vom Typ der Entwickler enthält.

11. Mittel zur Farbveränderung keratinischer Fasern, das unmittelbar vor der Anwendung durch Mischen einer ersten Komponente, enthaltend mindestens eine Entwicklerkomponente, mit einer zweiten Komponente, enthaltend mindestens ein Oxidationsmittel, erhalten wird, **dadurch gekennzeichnet, daß** es mindestens 0,1 Gew.-%, bezogen auf die Anwendungszubereitung, einer gegebenenfalls hydratisierten SiO₂-Verbindung in Form von Kieselgelen (Silicagel) oder als Wasserglas und mindestens einen strukturverbessernden Wirkstoff, der ausgewählt ist aus Panthenol, physiologisch verträglichen Panthenol-Derivaten, Mono-, Di- und Oligosacchariden, Serin, Niacinamid, Polyvinylpyrrolidon, Gluconsäure, Biotin und den lipidlöslichen Esteralkoholen oder Esterpolyolen, enthält.

12. Mittel nach einem der Ansprüche 6 bis 11, **dadurch gekennzeichnet, daß** die gegebenenfalls hydratisierte SiO₂-Verbindung ein Silicagel ist.

13. Mittel nach einem der Ansprüche 6 bis 11, **dadurch gekennzeichnet, daß** die gegebenenfalls hydratisierte SiO₂-Verbindung eine wäßrige Lösung eines Silikats der Formel (SiO₂)ₙ(Na₂O)ₘ(K₂O)ₚist, wobei n steht für eine positive rationale Zahl und m und p stehen unabhängig voneinander für eine positive rationale Zahl oder für 0, mit den Maßgaben, daß mindestens einer der Parameter m oder p von 0 verschieden ist und das Verhältnis zwischen n und der Summe aus m und p zwischen 1:4 und 4:1 liegt.

14. Mittel nach einem der Ansprüche 9 bis 13, **dadurch gekennzeichnet, daß** es als Farbstoffvorprodukt ferner mindestens eine Kupplerkomponente enthält.

15. Verfahren zur Blondierung keratinischer Faserm, **dadurch gekennzeichnet, daß** die Fasern mit einem Mittel nach einem der Ansprüche 6 bis 14 behandelt werden.

16. Verfahren zur oxidativen Färbung keratinischer Fasern, **dadurch gekennzeichnet, daß** die Fasern mit einem Mittel nach einem der Ansprüche 10 bis 14 behandelt werden.

## Claims

1. Use of optionally hydrated SiO₂ compounds for reducing damage to keratin fibres, more particularly human hair, by oxidative processes.

2. Use as claimed in claim 1, **characterized in that** the optionally hydrated SiO₂ compound is a silica gel.

3. Use as claimed in any of claims 1, **characterized in that** the optionally hydrated SiO₂ compound is an aqueous solution of a silicate with the formula (SiO₂)ₙ(Na₂O)ₘ(K₂O)ₚ, where n is a positive rational number and m and p independently of one another stand for a positive rational number or for 0, with the provisos that at least one of the parameters m or p is not 0 and the ratio between n and the sum of m and p is between 1:4 and 4:1.

4. Use claimed in any of claims 1 to 3, **characterized in that** the oxidative process is blonding.

5. Use claimed in any of claims 1 to 3, **characterized in that** the oxidative process is an oxidative hair colouring process.

6. Preparation for treating keratin fibres containing an oxidizing agent, at least one optionally hydrated SiO₂ compound in the form of silica gels or waterglass and at least one structure-improving active component selected from panthenol, physiologically safe panthenol derivatives, mono-, di- and oligosaccharides, serine, niacinamide, polyvinyl pyrrolidone, gluconic acid, biotin and the lipid-soluble ester alcohols or ester polyols.

7. Preparation as claimed in claim 6, **characterized in that** it contains at least 0.3% by weight of the optionally hydrated SiO₂ compound.

8. Preparation as claimed in claim 6 or 7, **characterized in that** it contains the structure-improving component in quantities of 0.1 to 5% by weight.

9. Preparation as claimed in any of claims 6 to 8, **characterized in that** it also contains a magnesium compound.

10. Preparation as claimed in any of claims 6 to 9, **characterized in that** it contains at least one dye precursor of the primary intermediate type.

11. Preparation for changing the colour of keratin fibres which is obtained immediately before application by mixing a first component containing at least one primary intermediate with a second component containing at least one oxidizing agent, **characterized in that** it contains at least 0.1% by weight, based on the preparation to be applied, of an optionally hydrated SiO₂ compound in the form of silica gels or waterglass and at least one structure-improving active component selected from panthenol, physiologically safe panthenol derivatives, mono-, di- and oligosaccharides, serine, niacinamide, polyvinyl pyrrolidone, gluconic acid, biotin and the lipid-soluble ester alcohols or ester polyols.

12. Preparation as claimed in any of claims 6 to 11, **characterized in that** the optionally hydrated SiO₂ compound is a silica gel.

13. Preparation as claimed in any of claims 6 to 11, **characterized in that** the optionally hydrated SiO₂ compound is an aqueous solution of a silicate with the formula (SiO₂)ₙ(Na₂O)ₘ(K₂O)ₚ, where n is a positive rational number and m and p independently of one another stand for a positive rational number or for 0, with the provisos that at least one of the parameters m or p is not 0 and the ratio between n and the sum of m and p is between 1:4 and 4:1.

14. Preparation as claimed in any of claims 9 to 13, **characterized in that** it also contains at least one secondary intermediate as a dye precursor.

15. Process for blonding keratin fibres, **characterized in that** the fibres are treated with the preparation claimed in any of claims 6 to 14.

16. Oxidative process for colouring keratin fibres, **characterized in that** the fibres are treated with the preparation claimed in any of claims 10 to 14.

## Revendications

1. Utilisation de composés de SiO₂ le cas échéant hydratés pour diminuer l'altération de fibres kératiniques, en particulier de cheveux humains, par des processus d'oxydation.

2. Utilisation selon la revendication 1, **caractérisée en ce que** le composé de SiO₂ le cas échéant hydraté est un silicagel.

3. Utilisation selon l'une quelconque des revendications 1, **caractérisée en ce que** le composé de SiO₂ le cas échéant hydraté est une solution aqueuse d'un silicate de formule (SiO₂)ₙ(Na₂O)ₘ(K₂O)ₚ, n représentant un nombre rationnel positif et m et p représentant, indépendamment l'un de l'autre, un nombre rationnel positif ou 0, à condition qu'au moins un des paramètres m ou p soit différent de 0 et que le rapport entre n et la somme de m et de p soit compris entre 1:4 et 4:1.

4. Utilisation selon l'une quelconque des revendications 1 à 3, **caractérisée en ce que** le processus d'oxydation est une décoloration.

5. Utilisation selon l'une quelconque des revendications 1 à 3, **caractérisée en ce que** le processus d'oxydation est une teinture oxydante des cheveux.

6. Agent pour le traitement de fibres kératiniques, contenant un oxydant, au moins un composé de SiO₂ le cas échéant hydraté sous forme de gels siliciques (silicagel) ou de verre soluble et au moins une substance active d'amélioration de la structure, choisie parmi le panthénol, les dérivés physiologiquement compatibles du panthénol, les monosaccharides, les disaccharides et les oligosaccharides, la sérine, le niacinamide, la polyvinylpyrrolidone, l'acide gluconique, la biotine et les esteralcools ou les esterpolyols solubles dans les lipides.

7. Agent selon la revendication 6, **caractérisé en ce qu'**il contient au moins 0,3% en poids du composé de SiO₂ le cas échéant hydraté.

8. Agent selon l'une quelconque des revendications 6 ou 7, **caractérisé en ce qu'**il contient la substance d'amélioration de la structure en des quantités de 0,1 à 5% en poids.

9. Agent selon l'une quelconque des revendications 6 à 8, **caractérisé en ce qu'**il contient en outre un composé du magnésium.

10. Agent selon l'une quelconque des revendications 6 à 9, **caractérisé en ce qu'**il contient au moins un précurseur de colorant du type agent de développement.

11. Agent pour la modification de la teinte de fibres kératiniques qui est obtenu immédiatement avant l'utilisation par mélange d'un premier composant, contenant au moins un composant de développement, avec un deuxième composant, contenant au moins un oxydant, **caractérisé en ce qu'**il contient au moins 0,1% en poids, par rapport à la préparation d'utilisation, d'un composé de SiO₂ le cas échéant hydraté sous forme de gels siliciques (silicagel) ou de verre soluble et au moins un agent d'amélioration de la structure, choisi parmi le panthénol, les dérivés physiologiquement compatibles du panthénol, les monosaccharides, les disaccharides et les oligosaccharides, la sérine, le niacinamide, la polyvinylpyrrolidone, l'acide gluconique, la biotine et les esteralcools ou les esterpolyols solubles dans les lipides.

12. Agent selon l'une quelconque des revendications 6 à 11, **caractérisé en ce que** le composé de SiO₂ le cas échéant hydraté est un silicagel.

13. Agent selon l'une quelconque des revendications 6 à 11, **caractérisé en ce que** le composé de SiO₂ le cas échéant hydraté est une solution aqueuse d'un silicate de formule (SiO₂)ₙ(Na₂O)ₘ(K₂O)ₚ, n représentant un nombre rationnel positif et m et p représentant, indépendamment l'un de l'autre, un nombre rationnel positif ou 0, à condition qu'au moins un des paramètres m ou p soit différent de 0 et que le rapport entre n et la somme de m et de p soit compris entre 1:4 et 4:1.

14. Agent selon l'une quelconque des revendications 9 à 13, **caractérisé en ce qu'**il contient en outre comme précurseur de colorant au moins un composant de couplage.

15. Procédé pour la décoloration de fibres kératiniques, **caractérisé en ce que** les fibres sont traitées avec un agent selon l'une quelconque des revendications 6 à 14.

16. Procédé pour la teinture oxydante de fibres kératiniques, **caractérisé en ce que** les fibres sont traitées avec un agent selon l'une quelconque des revendications 10 à 14.
